(19) Europäisches Patentamt
European Patent Office
Office européen des brevets

(11) Publication number : 0 101 651 B2

(12) NEW EUROPEAN PATENT SPECIFICATION

(45) Date of publication of the new patent specification : 31.03.93 Bulletin 93/13

(51) Int. Cl.[5] : C07C 15/02, C07C 2/78

(21) Application number : 83304805.1

(22) Date of filing : 19.08.83

# (54) Process for highly selective production of polyalkylbenzenes.

The file contains technical information submitted after the application was filed and not included in this specification

(30) Priority : 20.08.82 JP 144383/82

(43) Date of publication of application : 29.02.84 Bulletin 84/09

(45) Publication of the grant of the patent : 05.03.86 Bulletin 86/10

(45) Mention of the opposition decision : 31.03.93 Bulletin 93/13

(84) Designated Contracting States : FR GB NL

(56) References cited :
EP-A- 0 012 514
EP-A- 0 051 318
EP-A- 0 094 693
GB-A- 1 446 522
US-A- 2 945 899
US-A- 4 238 630

(56) References cited :
W.R. Moser, "Catalysis of Organic Reactions", Marcel Dekker Inc., New York (1981), pages 73-94, "Molecular shape-selective catalysis in zeolites", (Chang et al.)
Journal of Catalysis 47 (1977), pages 249-259
Journal of Catalysis 56 (1979), pages 169-173
CHEMICAL ABSTRACTS, vol. 88, no. 3, 16th Jan. 1978, Columbus, Ohio, US; K. KOGUCHI et al.: "Production of durene by methylation of 1,2,4-trimethylbenzene with methanol over zeolite catalyst", page 578, abstract no. 22 247e

(73) Proprietor : TOA NENRYO KOGYO KABUSHIKI KAISHA
1-1 Hitotsubashi, 1-Chome Chiyoda-Ku
Tokyo 100 (JP)

(72) Inventor : Maejima, Tsugio
1428-34, Oaza-Suneori Tsurugashima-cho
Iruma-gun Saitama-ken (JP)
Inventor : Tagaya, Nobuaki
2735-4, Oaza Kasahata
Kawagoe-shi Saitama-ken (JP)
Inventor : Sakurada, Satoshi
745-17, Sashiogi
Omiya-shi Saitama-ken (JP)

(74) Representative : Northover, Robert Frank et al
ESSO Chemical Limited Esso Chemical Research Centre P.O. Box 1
Abingdon Oxfordshire, OX13 6BB (GB)



Jouve, 18, rue Saint-Denis, 75001 PARIS

## Description

This invention relates to a process for selectively producing 1,2,4,5-tetraalkylbenzenes by alkylating raw material containing 1,2,4-trialkylbenzenes as the principal constituents.

Alkylation of aromatic hydrocarbon compounds with an alkylating agent such as alcohol or an olefin in the presence of a certain crystalline zeolite catalyst is known. Csicsery summarized catalytic alkylation of benzene, toluene or the like with an olefin, an alcohol or the like on Y-type or mordenite zeolite (cf. Robo, "Zeolite Chemistry and Catalysis", ACS Monograph, *171*, 1976, Chapter 12). Further, EP-A-0012514 discloses alkylation of benzene, toluene, ethylbenzene, isopropylbenzene, o-xylene and chlorobenzene with methyl alcohol or an olefin of 2 to 5 carbon atoms in the presence of a specified type of zeolite catalyst under a liquid phase condition. The fact that the alkylation reaction itself of aromatic hydrocarbon compounds is promoted in the presence of an acidic catalyst is well-known to those skilled in the art. Japanese Patent Publication No. 26528/1975 discloses a process for producing polymethylbenzenes by alkylating lower polymethylbenzenes of 8 to 9.5 carbon atoms with methyl chloride by using an aluminum chloride catalyst.

In these prior art processes, however, the formed polyalkylbenzenes, particularly tetramethylbenzenes of 10 carbon atoms are by-products which are present in small amounts, or even if they are main products, they are present as an isomeric mixture of a composition approximately in a thermodynamic equilibrium, so that they can only serve as a raw material which is markedly disadvantageous in the industry. More particularly, isomers of tetramethylbenzenes include a 1,2,4,5-substituted isomer (durene), a 1,2,3,5-substituted isomer (isodurene) and a 1,2,3,4-substituted isomer (prehnitene) and durene is in the greatest demand among them, because it is a valuable product which is particularly useful in the production of pyromelitic acid which is an intermediate for the production of polyimide resins. On the other hand, the separation of durene from an isomeric mixture of tetramethylbenzenes requires expensive superfractionation and multi-stage refrigeration steps and such processes, as will be realized, involve high operation cost and have a limited yield.

Description will now be made of the prior art processes from the viewpoint of the selective production of durene (1,2,4,5-tetramethylbenzene) which is the principal effect of this invention. Chemical Abstracts, vol. 88, No. 3, 16, describes the production of durene by methylation of 1,2,4-trimethylbenzene with methanol over a zeolite catalyst. Conversions of 70 mol % are described giving only 19 mol % durene. In Japanese Patent Laid-Open No. 76027/1975, Stephen Allan Butter et al disclosed a process for obtaining a product containing aromatic hydrocarbons by catalytic conversion of raw material containing aliphatic oxygen-containing organic compounds. This process differs from the present invention in requirements for catalyst constitution, operating conditions and product distribution, in that the product is a mixture comprising at least aliphatic and aromatic hydrocarbons and tetramethylbenzenes constituting a portion of the aromatic product which is rich in durene isomer. Moreover, their process necessitates the use of a catalyst containing a crystalline aluminosilicate zeolite having a silica/alumina ratio of at least 12 and a constraint index of 1 to 12 and having pore windows comprising 10-membered oxygen rings in the framework. To increase the proportion of formed durene in the tetramethylbenzene product in this process, a methyl group-containing compound is used and catalytic conversion must be carried out at a temperature of 550 to 850°F (288°C to 454°C) under an elevated pressure, (20 to 200 atm). The formation of aliphatic and aromatic hydrocarbon compounds which correspond to the gasoline fraction from the methyl group-containing compound, for example, methanol, is generally considered to proceed according to the following scheme, but detailed elementary reaction processes and their relation with catalysis in this catalytic conversion have not yet been elucidated.

$2\ CH_3OH \rightleftarrows CH_3OCH_3 + H_2O$

↓

light olefin ($C_2$ to $C_5$)+$H_2O$

↓ ↑

$C_5$+ +olefin

↓

aliphatic hydrocarbons

aromatic hydrocarbons

This invention provides a process for the highly selective production of 1,2,4,5-tetraalkylbenzenes by alkylation of aromatic hydrocarbon raw material containing 1,2,4-trialkylbenzenes as the principal constituents, which employs a particular form of a certain zeolite catalyst which not only permits the above-mentioned alkylation reaction to be effected with yields which are of industrial interest but also acts so as to yield a product with higher selectivity to durene which is of more industrial interest.

Namely, this invention relates to the selective production of polyalkylbenzenes comprising, a alkylating a 1,2,4-substituted isomer of trialkylbenzenes, which may be in the form of a hydrocarbon raw material containing said isomer in an amount by weight which is larger than those of any other constituents, with an alkylating agent by contacting with a catalyst under conversion conditions, said catalyst comprising a crystalline aluminosilicate zeolite which has an adsorption capacity of at least 2 ml per 100-g crystalline aluminosilicate zeolite for alkylbenzene molecules of a minimum molecular diameter of 7 to 8 Å and shows a shape-selective adsorptivity for alkylbenzenes of the minimum molecular diameter.

The process of this invention comprises a process for preparing 1,2,4,5-tetramethylbenzene by alkylating 1,2,4-trimethylbenzenes with methanol in the presence of a crystalline aluminosilicate zeolite, at a temperature of 280 °C under atmospheric pressure, said crystalline aluminosilicate zeolite being characterized in that the adsorption capacity (as defined above) is at least 2 ml/100 g-zeolite and the shape-selection coefficients or shape selectivity on the basis of the adsorption capacity for 1,2,4-trimethylbenzene are greater that 1.1 for p-xylene over 1,2,4-trimethylbenzene and smaller than 1.0 for 1,2,3-trimethylbenzene over 1,2,4-trimethylbenzene, as measured under conditions in which p-xylene, 1,2,4-trimethylbenzene and 1,2,3-trimethylbenzene coexist.

The process of this invention is carried out by contacting the above-mentioned mixture in the form of a continuous stream with the catalyst at a 1,2,4-trialkylbenzene/alkylating agent mol ratio of 1/2 and desirably a WHSV (weight hourly space velocity) of 0.1 to 100, preferably 1 to 10. In the operation of this invention, the mixture of an alkylating agent and aromatic hydrocarbon raw material can be in the form of gas, liquid or mixed phase. Although it is not necessarily essential, desirable alkylation is carried out preferably in the presence of hydrogen. Hydrogen can be used in the alkylation zone in an amount of from 0 to 10, preferably from 0 to 5 mol per mol said mixture.

Preferable zeolites are of TSZ and ZSM-5 types as described in more detail hereinafter, usually as a composite with a binder such as alumina, silica or an alkaline earth metal.

The zeolites which can be used in this invention are characterized by the adsorption capacity and the shape-selection coefficient which will be described hereinbelow in detail, and this is a fact which has been found as a result of studies and experiments by the inventors of this invention. Such zeolites, both natural and synthetic products, have reticular crystal lattices composed of $TO_4$ tetrahedra, wherein T is silicon (SI) and aluminum (Al), for the most part, and O represents oxygen, which are bonded together so as to share one oxygen atom among them. This manner of bonding determines the framework of a zeolite and zeolites are classified into a variety of types from the viewpoint of the structure according to the difference in this manner of bonding.

In the network structure of connected $TO_4$ tetrahedra, namely in the framework, cavities or channels consisting of consecutive cavities are formed, and the water of crystallization and cations are incorporated therein. When this hydrated zeolite is heated, it liberates the water of crystallization without damage to its framework structure to form a porous body. The dehydrated zeolite may adsorb various molecules through micropores, "pore windows", into cavities or channels. These pore windows have a uniform diameter of usually several Å, so that they can provide a so-called molecular sieve effect which permits adsorption of only those molecules that have a molecular diameter smaller than the diameter of the pore windows. Of course, the diameter of pore windows, varies with the difference in the framework structures of zeolites. Moreover, in case of zeolites of the same framework structure, the diameter of pore windows differs also with the extent of liberation of the water of crystallization, the difference in kind and amount of ions which are present within cavities and channels, and the difference in kind and ratio (a ratio expressed generally by a silica/alumina ratio) of elements (T) which form the framework.

Based on these scientific foundations, it is possible to vary the diameter of pore windows and the volume of cavities and channels by the techniques of zeolite synthesis and modification such as acid treatment, ion exchange treatment and heat-treatment.

The diameter of pore windows of zeolite is generally expressed or designated by a crystallographic micropore diameter or an effective micropore diameter. The former is the diameter of a pore window determined by precise measurement of zeolite structure by means of X-ray crystal structure analysis to determine the number and special dimensions of T or O (oxygen) which form pore windows On the other hand, the latter is the diameter of a pore window obtained by allowing zeolite to adsorb various molecules having different molecular diameters, estimating an apparent value from the molecular diameter of the largest molecule adsorbed and using this apparent value. Although both the diameters coincide approximately with each other in some cases, the effective micropore diameter is measured in many cases, to be somewhat larger because there is, in adsorption, a possibility of deformation of molecules to be adsorbed which permits adsorption of molecules which have a molecular diameter larger than the crystallographic micropore diameter. On the other hand, in some zeolites, the effective micropore diameter is sometimes smaller than the crystallographical one because of disordered crystal lattices or plugging of pore windows by non-framework structural substances. To obtain a

desirable capability in the use of zeolite, however, it is possible to vary purposely the effective micropore diameter, adsorption capacity and hereafter-mentioned shape-selection coefficient of zeolite by producing disordered crystal lattices or introducing a non-framework structural substance by the techniques of zeolite synthesis and modification such as ion exchange, impregnation, physical mixing, heat-treatment and acid or alkali treatment.

In view of the fact that little is known as yet about the structures of zeolites, both natural and synthetic products, and that, when zeolite is applied to a catalyst as in the case of this invention, the adsorption of compounds present in the reaction system becomes an important factor, the effective micropore diameter, rather than the crystallographic one, provides a practical index.

It might be presumed that, in order that zeolite be effective in the highly selective production of a 1,2,4,5-tetraalkylbenzene by alkylation of an aromatic hydrocarbon raw material containing 1,2,4-trialkylbenzene as the principal constituents, zeolite must have such an effective micropore diameter as to permit adsorption of at least 1,2,4-trimethylbenzene. Although a hydrogen form or mere polyvalent metal ion forms of the synthetic zeolites designated as X type or Y type zeolites, have an effective micropore diameter sufficient to permit adsorption of 1,2,4-trialkylbenzene and actually exhibit such an ability, these types of zeolite are not effective in this invention. From this fact and the concepts mentioned hereinabove, it is apparent that the effective micropore diameter which represents the pore diameter of cavities and channels of zeolite can only determine the upper limit of the diameter of molecules which zeolite can adsorb, and therefore effective micropore diameter alone is insufficient to discriminate as to whether a zeolite is effective in the alkylation process.

As a result of various studies and experiments, the inventors have devised the process of invention in which a functional test is used to discriminate whether a zeolite is effective in this invention or not. This test also includes information derived from the qualitative phenomena that, when molecules having molecular diameters which are smaller than the effective micropore diameter of zeolite are adsorbed by zeolite under given conditions, the rate of adsorption usually becomes larger as the molecular diameter becomes smaller and as the affinity for the zeolite becomes larger, that the relation between the size of the micropore and that of the molecule influences the diffusion rate of the molecules into the micropores and that polar molecules have a higher rate of adsorption because of their particularly strong affinity, and the test can give quantitative indications about the adsorption capacity and selectively of zeolite for molecules to be adsorbed.

This test method is referred to as "Test for evaluation of molecular shape selectivity" which will be described below and it essentially consists in measuring the adsorption capacities for molecules to be adsorbed under conditions in which p-xylene (minimum molecular diameter 7.0 Å), 1,2,4-trimethylbenzene (minimum molecular diameter 7.6 Å) and 1,2,3-trimethylbenzene (minimum molecular diameter 8.1 Å) coexist in a liquid phase and these molecules are competitively adsorbed by the zeolite, and using "total adsorption capacity" and "shape-selection coefficient" derived from the above adsorption capacity as parameters for the above-mentioned discrimination. The values of these parameters for several zeolites are shown below.

| Zeolite | Total adsorption capacity ml/100 g | Shape-selection coefficient | |
|---|---|---|---|
| | | [p-X/1,2,4-TMB] | [1,2,3-TMB]/[1,2,4-TMB] |
| TSZ | 7—12 | 2—15 | 0.9—0.4 |
| ZSM-5 | 7—13 | 2—14 | 0.9—0.5 |
| ferrierite | 2—8 | 3—10 | 0.9—0.6 |
| offretite | 6—11 | 1.5—3 | 1—0.8 |
| mordenite | 6—10 | 1.2—2 | 1—0.8 |
| Y | 15—19 | 1—1 | 1.1—1 |
| X | 15—20 | 1—1.1 | 1.1—1 |
| A | 0—1 | More than 10 | Less than 1 |

In interpreting the test method and significance of the total adsorption capacity and shape-selection coefficient, account must be taken of a possibility that, when a given zeolite is subjected to tests under somewhat different conditions, the values of the parameters vary. The total adsorption capacity and the shape-selection coefficient somewhat vary according to the concentration of the three kinds of molecules to be adsorbed, the

temperature of adsorption operation, the form of zeolite, i.e., powder or moldings, the presence or absence of binders and the like, in the test for evaluating the molecular shape selectivity. Furthermore, if 1,2,4-trimethylbenzene is replaced with o- or m-xylene having the same minimum molecular diameter (7.6 Å), the values of the parameters will vary. Therefore, even when the total adsorption capacity or shape-selection coefficient of a certain zeolite is different from the one mentioned above, such a zeolite has to be included within the scope of the catalysts effective in this invention, as far as its measurement is included within the concepts of the test method of this invention.

In the present invention effective catalysts are zeolites having a total adsorption capacity of at least 2 ml/100 g-zeolite, a shape-selection coefficient [p-X/1,2,4-TMB] of greater than 1.1 and a shape-selection coefficient [1,2,3-TMB/1,2,4-TMB] of lower than 1. Preferably, the catalysts are zeolites having a total adsorption capacity of at least 5 ml/100 g-zeolite, a shape-selection coefficient [p-X/1,2,4-TMB] of 1.5 to 10 and a shape-selection coefficient [1,2,3-TMB/1,2,4-TMB] of 0.9 or less.

Examples of a preferable type of zeolite are TSZ and ZSM-5 types which can be identified by the chemical composition and the X-ray diffraction data as described in Japanese Patent Application No. 143396/1981 and United States Patent No. 3,702,886.

The TSZ described herein refers to a crystalline aluminosilicate which has a particular framework structure belonging to the monoclinic system and in which the mol ratio, $SiO_2/Al_2O_3$, which represents the proportion of $TO_4$ tetrahedra forming the framework is relatively large, and it can be produced from an aqueous reaction mixture consisting essentially of inorganic reaction materials comprising a silicon compound, an aluminium compound, an alkali metal compound and water. In case of the TSZ thus produced, it is possible to convert the contained alkali metal directly into the hydrogen (H)-form or ammonium ($NH_4$)-form which is a precursor thereof and form of other metals, by an ion exchange operation.

According to the structure analysis by G. T. Kokotailo (Nature, *272*, 437 (1978)), ZSM-5 is a zeolite having an orthorhombic framework and usually it can be produced form an aqueous reaction mixture containing an organic nitrogen compound. In case of the thus-produced ZSM-5 in the form as it is produced, free space within the crystal is occupied by ions of the organic nitrogen compound as a starting material, so that it is impossible to convert such ZSM-5 directly into other forms as different from the case of TSZ and can be converted into other forms only when it is subjected to treatments such as heating and calcination, to remove said organic compound. This fact explicitly shows that TSZ is more advantageous than ZSM-5 from the viewpoint of availability by the actual production of zeolite catalysts.

On occasion, it is also possible to convert natural and synthetic zeolites other than those mentioned above into catalysts effective in this invention by treatments such as ion exchange, acid or alkali treatment, aluminum extraction, steam treatment, calcination, impregnation, and physical mixing and combinations of a variety of procedures for activation. Examples of zeolites which are convertible in this manner include ferrierite, brewsterite, stilbite, dachiardite, heulandite, epistilbite, clinoptilolite, gmelinite, mordenite and offretite.

When zeolite is synthesized in the form containing alkali cations, it is preferable to convert if into the hydrogen form so as to use it as the catalyst of this invention. Although the conversion into the hydrogen form can be effected by an acid treatment, it is generally carried out by conversion into the ammonium form as a precursor by ammonium ion exchange and thereafter calcination the ammonium form to yield the hydrogen form finally. In addition to the hydrogen form, zeolites of a form prepared by removing part or all of the alkali cations which are originally present in the zeolite can also be used. Namely, it is also possible to use zeolites which are subjected to ion exchange treatments so that the alkali cations are replaced with other suitable ions of Group IB to VIII metals of the periodic table, such as nickel, copper, zinc, palladium, magnesium or rare earth metals.

It is also possible to use the zeolites of these forms after they have been converted into forms effective in this invention by application of a combination of a variety of treatments such as acid or alkali treatment, steam treatment, baking, or introduction of metals or metal oxides as non-framework structural substances.

In this invention, synthetic zeolites have a mol ratio, $SiO_2/Al_2O_3$, preferably in the range of from 10 to 200 and more suitably in the range of from 10 to 80. Usually, synthetic zeolites have crystal particle diameters of from less than 1 micron to several tens of microns and these zeolites can be used as the catalysts of this invention directly or after a treatment of particle size adjustment.

To carry out a desirable alkylation process, it is desirable to incorporate zeolite into a substance which is resistant to temperatures and other conditions used in said process. In fact, in the practice of the process of this invention, it is advantageous to use a zeolite which is in the form of a composite with a substance which is catalytically relatively inactive in the alkylation reaction. The suitable substance is an inorganic oxide, and a particularly suitable substance is alumina which is not contaminated with other substances of this kind, and it is possible to use a zeolite in the form of a composite containing such a substance in an amount ranging from 5 to 70% by weight.

The aromatic hydrocarbon raw material which can be used in the process of this invention may be any of those which contain 1,2,4-trialkylbenzenes as the principal constituents and, preferably, 1,2,4-trialkylbenzene themselves and fractions obtained from petroleum of coal hydrocarbons and containing 1,2,4-trialkylbenzenes as the principal constituents. Generally, the feed contains the 1,2,4-trialkylbenzenes in a weight amount greater than any other hydrocarbon constituent in the feed. Naturally, aromatic hydrocarbons formed in the reforming or cracking of naphtha are suitable. Also it is possible to use trialkylbenzenes which are alkylated derivatives of benzene, toluene and xylene.

The process of this invention can be carried out under conditions in which a raw material mixture containing aromatic hydrocarbons and an alkylating agent is in a gas or liquid phase or a mixed phase of the both. The process of this invention can be carried out in any of batchwise, semicontinuous and continuous operation form by using one of or a combination of fixed bed, fluidized bed and moving bed catalyst systems.

The following examples are to illustrate the process of this invention.

Test method for evaluation of molecular shape selectivity

It has been recognized that the measured amounts of adsorption of various compounds having different molecular diameters effectively serve as parameters for discriminating zeolites which can be used in this invention. Namely, under conditions of liquid-phase competitive adsorption in which three compounds to be adsorbed, i.e., p-xylene (minimum molecular diameter 7.0 Å), 1,2,4-trimethylbenzene (minimum molecular diameter 7.6 Å) and 1,2,3-trimethylbenzene (minimum molecular diameter 8.1 Å) coexist in a 1,3,5-triisopropylbenzene solvent, the adsorption capacity for each kind of molecule is measured and specific values of the adsorption capacities of the molecules derived from the measurement can be used as the parameters.

A standard test method employed will now be described. A liquid mixture of 1,3,5-triisopropylbenzene and 3 vol. % of each of p-xylene, 1,2,4-trimethylbenzene and 1,2,3-trimethylbenzene is prepared. A predetermined amount of the mixture is placed in a sealable container and then a zeolite which has been calcined at 500 to 600°C and stored at room temperature under nonhygroscopic conditions is precisely weighed out and placed in the same container. The volume of the mixture at this time is adjusted to be 4 ml per g-zeolite. The sealed container containing the zeolite and the mixture is kept for 20 hours in an atmosphere of a constant temperature of 40°C and then a very small amount of sample is taken out from the liquid phase portion and quantitatively analyzed for each compound. The analysis is carried out on a 0.5-yl liquid phase sample by means of a gas chromatograph equipped with a hydrogen flame ionization detector.

On the basis of the concentrations (in terms of volume which can be determined from the molecular weight and density of each compound) of p-xylene, 1,2,4-triethylbenzene, 1,2,3-trimethylbenzene and 1,3,5-triisopropylbenzene which are obtained by the analysis, the adsorption capacity for each of p-xylene, 1,2,4-trimethylbenzene and 1,2,3-trimethylbenzene is determined from the difference in the concentration valued before the adsorption operation (the above mixture alone is analyzed) and after the adsorption operation. In this test, an assumption is made that the amount of 1,3,5-triisopropylbenzene present in the liquid phase portion does not change in the course of the adsorption operation.

Respective values which are cited in the claims, detailed description, examples, etc., of this invention are defined as follows:

total adsorption capacity:

sum total of amounts of adsorbed p-xylene, 1,2,4-trimethylbenzene and 1,2,3-trimethylbenzene (ml/100 g-zeolite)

shape-selection coefficient [p-X/1,2,4-TMB]:

adsorption capacity of p-xylene/adsorption capacity of 1,2,4-trimethylbenzene

shape-selection coefficient [1,2,3-TMB/1,2,4-TMB]:

adsorption capacity of 1,2,3-trimethylbenzene/adsorption capacity of 1,2,4-trimethylbenzene.

Example 1 (preparation of the catalyst)

TSZ was prepared in the following manner. A solution was prepared by dissolving 21 g of aluminum sulfate ($Al_2(SO_4)_3 \cdot 17\ H_2O$) in 650 g of deionized water and further adding 18.5 g of concentrated sulfuric acid (95 wt.%) and 80 g of sodium chloride. This solution was mixed, under stirring, with a solution containing 100 g of deionized water and 270 g of water glass ($Na_2O$ 9.4 wt.%, $SiO_2$ 29.4 wt.%) (JIS No. 3 water glass) to obtain an aqueous reaction mixture having a composition, represented by a mol ratio of oxides of

$$4.1\ Na_2O \cdot Al_2O_3 \cdot 41\ SiO_2 \cdot 1590\ H_2O.$$

In this case, the mol ratio, $Cl^-/SiO_2$, of sodium chloride which was used as a mineralizer was 1.03. The aqueous reaction mixture was placed in a stainless (SUS) autoclave, heated and maintained at 180°C under an autogeneous pressure for 20 hours. The solid product crystallized was separated by filtration, washed with water and then dried at 110°C. This crystalline product was analyzed by powder X-ray diffractometry and identified as a TSZ crystal.

Moreover, chemical analysis of this crystalline product revealed that it had a chemical composition of 2.35 wt.% $Na_2O$, 4.01 wt.% $Al_2O_3$, 89.6 wt.% $SiO_2$, and 4.0 wt.% $H_2O$. This composition corresponded to the following mol ratio when represented by a mol ratio based on $Al_2O_3$:

$$0.96\ Na_2O \cdot Al_2O_3 \cdot 38\ SiO_2 \cdot 5.7\ H_2O$$

Example 2 (comparative)

A portion of the TSZ of Example 1 was calcined in air at 600°C for 3 hours and used as a catalyst. As a result of the test for evaluation of molecular shape selectivity, this catalyst had a total adsorption capacity of 8.4 ml/100 g-TSZ, a shape-selection coefficient [p-X/1,2,4-TMB] of 2.4 and a shape-selection coefficient [1,2,3-TMB/1,2,3-TMB] of 0.7. A raw material having a 1,2,4-trimethylbenzene/methanol mol ratio of 1/1 was passed on the catalyst at 350°C and a weight liquid hourly space velocity (WHSV) of 2.5 under atmospheric pressure. The conversion of 1,2,4-trimethylbenzene was 0.9 mol % and the proportion (% selectivity of produced tetramethylbenzene) was 73%. Further, the conversion of 1,2,4,5-tetramethylbenzene (durene) in the total tetramethylbenzenes was 99% or more.

Example 3

30 g of the TSZ prepared in Example 1 was subjected to an ion exchange treatment at 80°C four times (the length of time for each treatment was 1.5 hours) by using 15 ml of a 5-wt.% ammonium chloride solution per g-TSZ in each case. Next, the ion-exchanged product was washed with water, dried at 110°C and calcined in air at 550°C for 3 hours to prepare H (hydrogen form)-TSZ. This H-TSZ contained 0.02 wt.% $Na_2O$. Further, as a result of the test for evaluation of molecular shape selectivity, the H-TSZ had a total adsorption capacity of 8.7 ml/100 g-H-TSZ, a shape-selection coefficient [p-X/1,2,4-TMB] of 2.4 and a shape-selection coefficient [1,2,3-TMB/1,2,4-TMB] of 0.8. 1,2,4-Trimethylbenzene was alkylated with methanol at 280°C under atmospheric pressure by using the H-TSZ as a catalyst. The results are shown in Table 1.

The main (alkylated) products were tetramethylbenzenes of which 98.5% was accounted for by the 1,2,4,5-isomer. 1,2,4,5-Tetramethylbenzene (durene) is of industrial importance and, for instance, it is used for the production of pyromellitic acid which is an intermediate for the production of polyimide resins.

TABLE 1
Alkylation of 1,2,4-trimethylbenzene with methanol

| | |
|---|---|
| raw material | 1,2,4-trimethylbenzene/methanol=1/2 (mol ratio) |
| catalyst | H-TSZ |
| temperature | 280°C |
| pressure | atmospheric |
| WHSV | 2.5 (based on total materials) |
| conversion | 15% trimethylbenzene, >95% methanol |

| Products | Selectivity (mol %) | Isomer distribution (%) | |
|---|---|---|---|
| toluene | 1.2 | | |
| $C_8$ | 11.0 | p-xylene | 34 |
| | | m-xylene | 54 |
| | | o-xylene | 12 |
| $C_9$ | 4.1 | 1,3,5-trimethylbenzene | 43 |
| | | 1,2,3-trimethylbenzene | 13 |
| | | ethyltoluene | 44 |
| $C_{10}$ | 74.5 | 1,2,4,5-tetramethylbenzene | 98.5 |
| | | 1,2,3,5-tetramethylbenzene | 0.6 |
| | | 1,2,3,4-tetramethylbenzene | 0.9 |
| $C_{11}$ | 7.0 | pentamethylbenzene | 87 |
| $C_{12}^{+}$ | 2.2 | hexamethylbenzene | 65 |

Examples 4 through 8

1,2,4-Trimethylbenzene was alkylated with methanol in the presence of the H-TSZ catalyst as used in Example 3 by using various temperatures, space velocities and materials. The results are shown in Table 2.

TABLE 2

| | Ex. 4 comparative | Ex. 5 comparative | Ex. 6 comparative | Ex. 7 comparative | Ex. 8 |
|---|---|---|---|---|---|
| raw material: 1,2,4-trimethylbenzene/methanol (mol ratio) | 1/2 | 1/2 | 1/1 | 2/1 | 1/2 |
| catalyst | H-TSZ | H-TSZ | H-TSZ | H-TSZ | H-TSZ |
| temperature (°C) | 250 | 310 | 280 | 280 | 280 |
| pressure | atmospheric | atmospheric | atmospheric | atmospheric | atmospheric |
| WHSV | 2.5 | 2.5 | 3.1 | 2.8 | 3.8 |
| conversion: trimethylbenzene (mol %) | 5 | 37 | 10 | 6 | 15.5 |
| Product selectivity (mol %) | | | | | |
| toluene | 0.1 | 1.9 | 1.9 | 2.0 | 1.4 |
| $C_8$ | 6.0 | 41.0 | 14.5 | 16.4 | 12.5 |
| $C_9$ | 1.2 | 6.4 | 7.6 | 10.5 | 5.9 |
| $C_{10}$ | 81.0 | 43.1 | 69.5 | 65.0 | 71.0 |
| $C_{11}$ | 9.6 | 6.2 | 4.7 | 2.9 | 7.6 |
| $C_{12}'$ | 2.1 | 1.4 | 1.8 | 3.2 | 1.6 |

TABLE 2 (contd.)

| Product isomer distribution (%) | | Ex. 4 | Ex. 5 | Ex. 6 | Ex. 7 | Ex. 8 |
|---|---|---|---|---|---|---|
| $C_8$ | p-xylene | 32 | 35 | 34 | 32 | 33 |
| | m-xylene | 57 | 51 | 52 | 50 | 55 |
| | o-xylene | 11 | 14 | 14 | 18 | 12 |
| $C_9$ | 1,3,5-trimethylbenzene | 70 | 45 | 61 | 67 | 43 |
| | 1,2,3-trimethylbenzene | 24 | 12 | 18 | 22 | 12 |
| | ethyltoluene | 6 | 43 | 21 | 11 | 45 |
| $C_{10}$ | 1,2,4,5-tetramethylbenzene | 99 | 91 | 98 | 94 | 99 |
| | 1,2,3,5-tetramethylbenzene | 0.4 | 5 | 1 | 4 | 0.4 |
| | 1,2,3,4-tetramethylbenzene | 0.6 | 4 | 1 | 2 | 0.6 |
| $C_{11}$ | pentamethylbenzene | 91 | 78 | 86 | 88 | 86 |
| $C_{12}$ | hexamethylbenzene | 79 | 59 | 62 | 77 | 67 |

Example 9 (preparation of the catalyst)

A TSZ sample was prepared in the following manner. A solution (solution A) was prepared by mixing 9.5 g of sodium aluminate containing $Al_2O_3$ and $Na_2O$ in amounts of 35.7 wt.% and 29. 1 wt.%, respectively, and 273 g of water glass ($Na_2O$: 9.4 wt.%, $SiO_2$: 29.4 wt.%) in 570 g of deionized water. Separately, a solution (solution B) was prepared by diluting 68 g of concentrated hydrochloric acid (35 wt.%) with 175 g of deionized water. Further, a sodium chloride solution was prepared by dissolving 48 g of sodium chloride in 400 g of deionized water. Solutions A and B were simultaneously added dropwise to this aqueous sodium chloride solution under agitation to obtain an aqueous reaction mixture having a composition, represented by a mol ratio of oxides:

$$4.0\ Na_2O \cdot Al_2O_3 \cdot 40\ SiO_2 \cdot 2272\ H_2O$$

This aqueous reaction mixture was placed in a SUS autoclave, and maintained, by heating, at 180°C for 24 hours under an autogeneous pressure. The obtained crystalline product was analyzed by powder X-ray diffractometry and identified as a TSZ crystal.

Further, in order to obtain a hydrogen-form TSZ, the TSZ was subjected to an ion exchange treatment, water washing and calcination treatment in the same manner as described in Example 3. This H-TSZ was subjected to a chemical analysis and the test for evaluation of molecular shape selectivity, and the following values were obtained.

| Composition | Evaluation of shape selectivity | |
|---|---|---|
| $Na_2O$ 0.02 wt.% | total adsorption capacity 9.1 ml/100 g-zeolite | |
| $Na_2O_3$ 5.29 wt.% | | |
| | Shape-selection coefficient | |
| $SiO_2$ 94.2 wt.% | p-X/1,2,4-TMB | 1.9 |
| $SiO_2/Al_2O_3$ 30.3 (mol ratio) | 1,2,3-TMB/1,2,4-TMB | 0.8 |

Example 10

1,2,4-Trimethylbenzene was alkylated with methanol at 280°C under atmospheric pressure by using the H-TSZ prepared in Example 9 as a catalyst. The operating conditions and the products obtained are shown in Table 3.

TABLE 3

| | |
|---|---|
| raw material | 1,2,4-trimethylbenzene/methanol=1/2 (mol ratio) |
| catalyst | H-TSZ (Ex. 9) |
| temperature | 280°C |
| pressure | atmospheric |
| WHSV | 2.5 |
| conversion | 22.5 mol % trimethylbenzene, >95 mol % methanol |

| Products | Selectivity (mol %) | Isomer distribution (%) | |
|---|---|---|---|
| toluene | 1.7 | | |
| $C_8$ | 8.5 | p-xylene | 31 |
| | | m-xylene | 60 |
| | | o-xylene | 9 |
| $C_9$ | 6.6 | 1,3,5-trimethylbenzene | 45 |
| | | 1,2,3-trimethylbenzene | 13 |
| | | ethyltoluene | 42 |
| $C_{10}$ | 78.5 | 1,2,4,5-tetramethylbenzene | 96.5 |
| | | 1,2,3,5-tetramethylbenzene | 2.0 |
| | | 1,2,3,4-tetramethylbenzene | 1.5 |
| $C_{11}$ | 4.1 | pentamethylbenzene | 83 |
| $C_{12}^+$ | 0.6 | hexamethylbenzene | 61 |

Example 11

In the presence of the same H-TSZ catalyst as that used in Example 3, a trimethylbenzene isomer mixture (30% 1,3,5-/60% 1,2,4-/10%, 1,2,3-trimethylbenzenes) having a composition approximately in a thermodynamical equilibrium was alkylated with methanol. The operating conditions and the products obtained are shown in Table 4.

The main products were tetramethylbenzenes of which 93% was accounted for by the 1,2,4,5-isomer (durene).

TABLE 4

| | | | |
|---|---|---|---|
| raw material | trimethylbenzene/methanol=1/2 (mol ratio) | | |
| catalyst | H-TSZ | | |
| temperature | 280°C | | |
| pressure | atmospheric | | |
| WHSV | 5.6 | | |
| conversion | 11.5 mol % trimethylbenzene, ≃90 mol % methanol | | |

| Products | Selectivity (mol %) | Isomer distribution (%) | |
|---|---|---|---|
| toluene | 1.2 | | |
| $C_8$ | 18.1 | p-xylene<br>m-xylene<br>o-xylene | 32<br>49<br>19 |
| $C_9$ | 1.1 | ethyltoluene | 100 |
| $C_{10}$ | 71.1 | 1,2,4,5-tetramethylbenzene<br>1,2,3,5-tetramethylbenzene<br>1,2,3,4-tetramethylbenzene | 93<br>3<br>4 |
| $C_{11}$ | 6.2 | pentamethylbenzene | 83 |
| $C_{12}^+$ | 2.3 | hexamethylbenzene | 62 |

Example 12

A 15-g portion was taken out from the TSZ prepared in Example 1 and subjected to an ion exchange treatment at 80°C four times (the length of time for each treatment was 2 hours) by using 13 ml of a 2-N aqueous magnesium chloride solution per g-TSZ in each case. Next, the ion-exchanged product was washed with water, dried at 110°C and calcined in air at 500°C for 3 hours to obtain a Mg (magnesium)-form TSZ. This Mg-form TSZ contained 0.03 wt.% $Na_2O$ and 1.6 wt.% MgO. The test for evaluation of molecular shape selectivity revealed that the Mg-TSZ had a total adsorption capacity of 8.2 ml/100 g-Mg-TSZ and a shape-selection coefficient [p-X/1,2,4-TMB] of 2.6 and a shape-selection coefficient [1,2,3-TMB/1,2,4-TMB] of 0.7.

1,2,4-Trimethylbenzene was alkylated with methanol at 280°C under atmospheric pressure by using this Mg-TSZ as a catalyst. The operating conditions and the products obtained are shown in Table 5.

TABLE 5

| | | | |
|---|---|---|---|
| raw material | 1,2,4-trimethylbenzene/methanol = 1/2 (mol ratio) | | |
| catalyst | Mg-TSZ | | |
| temperature | 280°C | | |
| pressure | atmospheric | | |
| WHSV | 3.8 | | |
| conversion | 19 mol % trimethylbenzene, >95 mol % methanol | | |
| Products | Selectivity (mol %) | Isomer distribution (%) | |
| toluene | 0.5 | | |
| $C_8$ | 13.6 | p-xylene<br>m-xylene<br>o-xylene | 2.3<br>68<br>9 |
| $C_9$ | 3.7 | 1,3,5-trimethylbenzene<br>1,2,3-trimethylbenzene<br>ethyltoluene | 46<br>13<br>41 |
| $C_{10}$ | 78.1 | 1,2,4,5-tetramethylbenzene<br>1,2,3,5-tetramethylbenzene<br>1,2,3,4-tetramethylbenzene | 98.5<br>0.7<br>0.8 |
| $C_{11}$ | 3.9 | pentamethylbenzene | 82 |
| $C_{12}^+$ | 0.2 | hexamethylbenzene | 76 |

Example 13

A hydrogen-form ZSM-5 was prepared in the following manner. A solution (solution A) was prepared by dissolving 5.4 g of aluminum sulfate in 145 g of deionized water and further adding thereto 14 g of concentrated sulfuric acid (95 wt.%) and 21 g of tetrapropylammonium bromide (TPA Br). Separately, a solution (solution B) was prepared by mixing 105 g of deionized water with 168 g of water glass ($Na_2O$: 9.4 wt.%, $SiO_2$: 29.4 wt.%). Further, an aqueous sodium chloride solution was prepared by dissolving 60 g of sodium chloride in 250 g of deionized water. The above-produced solutions A and B were simultaneously added dropwise to the aqueous sodium chloride solution under agitation to obtain an aqueous reaction mixture having a composition, represented by a mol ratio of oxides of

$$4.7\ (TPA)_2O \cdot 6.5\ Na_2O \cdot Al_2O_3 \cdot 99\ SiO_2 \cdot 4040\ H_2O.$$

This aqueous reaction mixture was placed in a SUS autoclave and maintained, by heating, at 160°C under an autogeneous pressure for 20 hours. The solid product crystallized was separated by filtration, washed with water and then dried at 110°C. As a result of an analysis by powder X-ray diffractometry, this crystalline product showed a diffraction pattern which agreed with that described in United States Patent No. 3,702,886, and was identified as ZSM-5.

25 g of the ZSM-5 thus produced was calcined in air at 540°C for 3 hours and then subjected to an ion exchange operation at 80°C four times by using 15 ml of a 5 wt.% ammonium chloride solution per g-zeolite in each case. The length of time for each operation was 1.5 hours. Then, the ion-exchanged product was thoroughly washed with water, dried at 110°C and calcined in air at 550°C for 3 hours to form an H (hydrogen)-ZSM-5. Chemical analysis of the H-ZSM-5 revealed that it had a composition of 0.01 wt.% $Na_2O$, 2.61 wt.% $Al_2O_3$, and 97.1 wt.% $SiO_2$. As a result of the test for evaluation of molecular shape selectivity, it was found that the H-ZSM-5 had a total adsorption capacity of 7.4 ml/100 g-H-ZSM-5, a shape-selection coefficient [p-X/1,2,4-TMB] of 2.2 and a shape-selection coefficient [1,2,3-TMB/1,2,4-TMB] of 0.6.

Example 14

1,2,4-Trimethylbenzene was alkylated with methanol at 280°C under atmospheric pressure by using the

H-ZSM-5 prepared in Example 13 as a catalyst. The operating conditions and the products obtained are shown in Table 6.

TABLE 6

| | |
|---|---|
| raw material | 1,2,4-trimethylbenzene/methanol=1/2 (mol ratio) |
| catalyst | H-ZSM-5 |
| temperature | 280°C |
| pressure | atmospheric |
| WHSV | 2.5 |
| conversion | 17.1 mol % trimethylbenzene, >95 mol % methanol |

| Products | Selectivity (mol %) | Isomer distribution (%) | |
|---|---|---|---|
| toluene | 0.9 | | |
| $C_8$ | 13.5 | p-xylene | 36 |
| | | m-xylene | 51 |
| | | o-xylene | 13 |
| $C_9$ | 3.4 | 1,3,5-trimethylbenzene | 44 |
| | | 1,2,3-trimethylbenzene | 12 |
| | | ethyltoluene | 44 |
| $C_{10}$ | 72.0 | 1,2,4,5-tetramethylbenzene | 99 |
| | | 1,2,3,5-tetramethylbenzene | 0.5 |
| | | 1,2,3,4-tetramethylbenzene | 0.5 |
| $C_{11}$ | 7.3 | pentamethylbenzene | 88 |
| $C_{12}^-$ | 2.9 | hexamethylbenzene | 70 |

Comparative Example A

1,2,4-Trimethylbenzene alone was reacted at 280°C and a WHSV of 2.5 under atmospheric pressure in the presence of the same H-TSZ catalyst as that used in Example 1. The results are shown in Table 7.

TABLE 7

| Conversion products | 4.6 Mol % trimethylbenzene selectivity (mol %) | Isomer distribution (%) | |
|---|---|---|---|
| toluene | 1.9 | | |
| $C_9$ | 60.4 | p-xylene | 29 |
| | | m-xylene | 50 |
| | | o-xylene | 21 |
| $C_9$ | 23.1 | 1,3,5-trimethylbenzene | 66 |
| | | 1,2,3-trimethylbenzene | 30 |
| | | ethyltoluene | 4 |
| $C_{10}$ | 9.5 | 1,2,4,5-tetramethylbenzene | 82 |
| | | 1,2,3,5-tetramethylbenzene | 13 |
| | | 1,2,3,4-tetramethylbenzene | 5 |
| $C_{11}$ | 5.1 | pentamethylbenzene | 90 |
| $C_{12}^-$ | 0 | | |

TABLE 8

| | | | |
|---|---|---|---|
| raw material | 1,2,4-trimethylbenzene/methanol=1/2 (mol ratio) | | |
| catalyst | H-Na-Y | | |
| temperature | 220°C | | |
| pressure | atmospheric | | |
| WHSV | 2.5 | | |
| conversion | 43.1 mol % trimethylbenzene, >95 mol % methanol | | |

| Products | Selectivity (mol %) | Isomer distribution (%) | |
|---|---|---|---|
| toluene | 0.4 | | |
| $C_8$ | 6.7 | p-xylene | 19 |
| | | m-xylene | 52 |
| | | o-xylene | 29 |
| $C_9$ | 9.4 | 1,3,5-trimethylbenzene | 62 |
| | | 1,2,3-trimethylbenzene | 29 |
| | | ethyltoluene | 9 |
| $C_{10}$ | 70.2 | 1,2,4,5-tetramethylbenzene | 43 |
| | | 1,2,3,5-tetramethylbenzene | 36 |
| | | 1,2,3,4-tetramethylbenzene | 21 |
| $C_{11}$ | 11.6 | pentamethylbenzene | 87 |
| $C_{12}^+$ | 1.7 | hexamethylbenzene | 71 |

Comparative Example B

Methanol alone was reacted under the same operating conditions as in Comparative Example A in the presence of the same catalyst as that used in Example 3. In this case, the conversion of methanol was about 90%, but the amounts of aromatic compounds formed was extremely small.

Comparative Example C

Sodium-form Y-type synthetic zeolite (Na-Y) (SK-40, a product of Union Carbide Corp.), dried at 120°C, was subjected to an ion exchange treatment. This treatment was carried out four times (each treating time was 2 hours) by using 15 ml of a 1-N aqueous ammonium chloride solution per g-Na-Y in each case. Then, the ion-exchanged product was washed with water, dried at 110°C and calcined in air at 500°C for 3 hours. As a result of a chemical analysis, it was found that the thus-produced Y-type zeolite had a mol ratio, $SiO_2/Al_2O_3$, of 4.8 and an exchange rate of sodium of 82%. The test for evaluation of molecular selectivity revealed that this zeolite had a total adsorption capacity of 17.8 ml/100 g-zeolite, a shape-selection coefficient [p-X/1,2,4-TMB] of 1.1 and a shape-selection coefficient [1,2,3-TMB/1,2,4-TMB] of 1.0.

1,2,4-Trimethylbenzene was alkylated with methanol at 220°C under atmospheric pressure by using the zeolite as a catalyst. The operating conditions and the products obtained are shown in Table 8.

The main products were tetramethylbenzenes, but this alkylation resulted in a relatively small selectivity for the 1,2,4,5-isomer.

The crystalline aluminosilicate zeolite, designated as TSZ, has the characteristic X-ray diffraction pattern shown in Table 9. The most important characterizing lines are followings:

(1) The line at about 14.7°2θ (d=6.03Å) is a singlet line.

(2) The doublet nature of the line at 23.0~23.3°θ (d=3.86~3.82Å) can clearly be seen (in the X-RD pattern).

(3)The X-ray diffraction patterns of TSZ show moloclinic characteristics.

TSZ can be distinguished from conventional zeolites in its the X-ray diffraction pattern. TSZ can also

be identified by the compositions, in synthesized form, expressed in mole ratios of oxides, as follows:

$$0.8 - 1.5\ Na_2O{:}Al_2O_3{:}10 - 100\ SiO_2{:}0 - 40\ H_2O$$

i.e. and alkaline cation is sodium and the silica/alumina mole ratio is from 10 to 100.

As-synthesized TSZ (Na form) materials can be converted to a catalytic materials for hydrocarbon conversion reactions by techniques well-known in the literature.

X-ray diffraction patterns of as-synthesized ZSM-5 materials, with containing an organic matter and a sodium cations, are consistent with orthorhombic crystal system. A change to monoclinic crystal system has been observed on certain treated materials, such as calcined ZSM-5 and its hydrogen form. But, TSZ can be distinguished from ZSM-5 in a silica/alumina ratio and a cation species. Tables 9, 10 and 11 show a comparison between TSZ and ZSM-5.

The method for preparing the crystalline aluminosilicate zeolite comprises preparing a reaction mixture consisting essentially in inorganic reagents and having a composition, in terms of mole ratios of oxides, within the following ranges:

$$SiO_2/Al_2O_3 = 10 \sim 100$$
$$Na_2O/SiO_2 = 0.03 \sim 0.5$$
$$H_2O/Na_2O = 100 \sim 1{,}000$$

and maintaining the mixture at an elevated temperature until the crystals of said aluminosilicate (zeolite) are formed.

TABLE 9

TSZ

X-ray powder diffraction pattern (with respect to significant lines)

| d (Å) | $I/I_o$ | d (Å) | $I/I_o$ |
|---|---|---|---|
| 11.2 ±.2 | S | 4.26±.07 | m |
| 10.1 } 9.9 } ±.2 | S | 3.86±.05 | VS |
| 9.7 ±.2 | S | 3.82±.05 | S |
| 7.5 ±.15 | m | 3.76±.05 | S |
| 7.1 ±.15 | VW | 3.70±.05 | S |
| 6.4 ±.1 | W | 3.64±.05 | S |
| 6.03 ±.1 | m | 3.05±.03 | m |
| 5.58 ±.1 | m | | |
| 5.03±.1 | W | | |
| 4.62 ±.07 | W | | |

Lattice constants of as-synthesized TSZ*

| | Monoclinic | Orthorhombic |
|---|---|---|
| a, Å | 20.159 (.004) | 20.161 (.005) |
| b, Å | 19.982 (.006) | 19.935 (.005) |
| c, Å | 13.405 (.005) | 13.437 (.007) |
| d, deg. | 90.51 (.03) | 90 |

*$Na_2O/Al_2O_3 = 1.02$ $SiO_2/Al_2O_3 = 26.2$

TABLE 10

Comparison of TSZ with ZSM-5

| | TSZ | ZSM-5 |
| --- | --- | --- |
| Preparation | Inorganic | with TPA salt |
| Symmetry | monoclinic | orthorhombic |
| Lattice constants | | |
| a (Å) | 20.159 | 20.07 (20.078) |
| b (Å) | 19.982 | 19.92 (19.922) |
| c (Å) | 13.405 | 13.42 (13.415) |
| β (beta) (deg.) | 90.51 | 90 |
| $SiO_2/Al_2O_3$ | 30—70 | 10—4,000 |
| Crystal size | large (μm) | small |
| Al distribution in crystals | uniform | Al zoning in rim |
| Binderless pellets (gel-preformed) | easy to prepare | hard |

TABLE 11

Crystal system of ZSM-5 zeolite

— As synthesized forms of ZSM-5 are assigned to be an orthorhombic crystal system.

— A change to monoclinic symmetry is observed upon certain treatments, such as calcination and ion-exchange.

| References | G. T. Kokotailo[1] | J. R. Anderson[2] | E. L. Wu[3] | TCRL data |
| --- | --- | --- | --- | --- |
| ZSM-5 | | | | |
| Forms | As-synthesized (Na, TPA) | Na-exchanged | Calcined H, $NH_4$-exchanged | As synthesized (Na, TPA) |
| $SiO_2/Al_2O_3$ (mol/mol) | Unknown | 27 | 70—3,000 | 47.8 |
| Crystal system | Orthorhombic | Orthorhombic | Monoclinic | Orthorhombic |
| Lattice constants | | | | |
| a, A | 20.07 | 20.119 | 20.11—20.17 | 20.078 |
| b, A | 19.92 | 19.925 | 19.90—19.94 | 19.922 |
| c, A | 13.42 | 13.419 | 13.40—13.43 | 13.415 |
| α, deg | — | — | 90.4 —90.6 | — |

[1]Nature 272, 30 Mar. 1978.
[2]J. Catalysis 58, 114, 1979.
[3]J. Phys. Chem. 83, 2777, 1979.

## Claims

1. A process for preparing 1, 2, 4, 5-tetramethylbenzene by alkylating 1, 2, 4-trimethylbenzene with methanol in the presence of a crystalline aluminosilicate zeolite which has adsorptive capacity of at least 2ml per

100g of zeolite for an alkylbenzene of a minimum molecular diameter of 7 to 8 A and which has a shape-selection coefficient for p-xylene over 1, 2, 4-trimethylbenzene of greater than 1.1 and a shape-selection coefficient for 1, 2, 3-trimethylbenzene over 1, 2, 4-trimethylbenzene of less than 1.0 at a mole ratio of 1, 2, 4-trimethylbenzene/methanol of 1/2, at 280°C under atmospheric pressure.

**2.** A process as claimed in claim 1 wherein the adsorptive capacity of the zeolite is at least 5ml per 100g of zeolite for alkylbenzenes of a molecular diameter of at least 7 to 8 A and the shape-selection coefficient of 1, 2, 3-trimethylbenzene over 1, 2, 4-trimethylbenzene of not greater than 0.9.

**3.** A process as claimed in claim 1 or claim 2, wherein the zeolite is TSZ or ZSM-5.

**4.** A process as claimed in any of claims 1 to 3, wherein the alkylation is carried out in the presence of hydrogen.

## Patentansprüche

**1.** Verfahren zur Herstellung von 1,2,4,5-Tetramethylbenzol durch Alkylierung von 1,2,4-Trimethylbenzol mit Methanol in Gegenwart eines kristallinen Aluminosilikatzeoliths mit einer Adsorptionskapazität von mindestens 2 ml je 100 g Zeolith für ein Alkylbenzol mit einem minimalen Molekulardurchmesser von 7 bis 8 Å und einem Formauswahlkoeffizienten für p-Xylol gegenüber 1,2,4-Trimethylbenzol von mehr als 1,1 und einem Formauswahlkoeffizienten von 1,2,3-Trimethylbenzol gegenüber 1,2,4-Trimethylbenzol von weniger als 1,0 bei einem Molverhältnis von 1,2,4-Trimethylbenzol/Methanol von 1/2 bei 280 °C unter Atmosphärendruck.

**2.** Verfahren nach Anspruch 1, bei dem die Adsorptionskapazität des Zeolithen mindestens 5 ml je 100 g Zeolith für Alkylbenzole mit einem Molekulardurchmesser von mindestens 7 bis 8 Å beträgt und der Formauswahlkoeffizient von 1,2,3-Trimethylbenzol gegenüber 1,2,4-Trimethylbenzol nicht mehr als 0,9 beträgt.

**3.** Verfahren nach Anspruch 1 oder 2, bei dem der Zeolith TSZ oder ZSM-5 ist.

**4.** Verfahren nach einem der Ansprüche 1 bis 3, bei dem die Alkylierung in Gegenwart von Wasserstoff durchgeführt wird.

## Revendications

**1.** Procédé de production de 1,2,4,5-tétraméthylbenzène par alkylation de 1,2,4-triméthylbenzène avec du méthanol en présence d'une zéolite du type aluminosilicate cristallin qui a une capacité d'adsorption d'au moins 2 ml pour 100 g de zéolite pour un alkylbenzène d'un diamètre moléculaire minimal de 7 à 8 Å et qui a un coefficient de sélection envers la forme pour le p-xylène par rapport au 1,2,4-triméthylbenzène supérieur à 1,1 et un coefficient de sélection envers la forme pour le 1,2,3-triméthylbenzène par rapport au 1,2,4-triméthylbenzène de moins de 1,0 pour un rapport molaire du 1,2,4-triméthylbenzène au méthanol de 1/2, à 280°C à la pression atmosphérique.

**2.** Procédé suivant la revendication 1, dans lequel la capacité d'adsorption de la zéolite est d'au moins 5 ml pour 100 g de zéolite pour des alkylbenzènes d'un diamètre moléculaire d'au moins 7 à 8 Å et un coefficient de sélection envers la forme du 1,2,3-triméthylbenzène par rapport au 1,2,4-triméthylbenzène n'excédant pas 0,9.

**3.** Procédé suivant la revendication 1 ou la revendication 2, dans lequel la zéolite est TSZ ou ZSM-5.

**4.** Procédé suivant l'une quelconque des revendications 1 à 3, dans lequel l'alkylation est conduite en présence d'hydrogène.